# FASCICULE DE BREVET EUROPEEN

(11) **EP 0 864 657 B1**
(45) Date de publication et mention de la délivrance du brevet: **27.08.2003**
(21) Numéro de dépôt: 98400595.9
(22) Date de dépôt: 13.03.1998
(51) Int. Cl.: C12Q 1/68

(54) **Oligonucléotides dérivés des gènes vts de bactéries E. coli productrices de vérotoxines et leurs utilisations**
Oligonukleotide von vts Genen aus Verotoxinen produzierenden E.coli und deren Verwendung
Oligonucleotides from vts genes of verotoxins producing E.coli and their use

(30) Priorité: 14.03.1997 FR 9703073
(43) Date de publication de la demande: 16.09.1998
(73) Titulaire: Centre National d'Etudes Vétérinaires et Alimentaires CNEVA, 94701 Maisons-Alfort Cedex (FR)
(72) Inventeur: Fach, Patrick, 94000 Creteil (FR); Perelle, Sylvie, 94220 Charenton Le Pont (FR); Dilasser, Françoise, 91290 La Norville (FR)
(74) Mandataire: Vialle-Presles, Marie José

(56) Documents cités:
- EP-A- 0 669 399
- WO-A-95/00664
- WO-A-96/30043
- CA-A- 2 078 716
- JP-A- 4 297 488
- JP-A- 7 008 280
- CALDERWOOD S . B. ET AL.,: "Nucleotid sequences of the Shiga-like toxin genes of E.coli" PROC. NATL. ACAD. SCI. USA, vol. 84, - juillet 1987 pages 4364-4368, XP002048513
- READ S. C. ET AL.,: "Polymerase chain reaction for detection of verotoxingenic E.coli isolated from animal and food sources" MOL. CELL. PROBES, vol. 6, - 1992 pages 153-161, XP002048514

### LISTE DE SEQUENCES

(1) INFORMATIONS GENERALES:
   (I) DEPOSANT:
      (A) NOM: CENTRE NATIONAL D'ETUDES VETERINAIRES ET ALIMENTAIRES - CNEVA
      (B) RUE: 23 AVENUE DU GENERAL DE GAULLE
      (C) VILLE: MAISONS ALFORT
      (E) PAYS: FRANCE
      (F) CODE POSTAL: 94701
   (II) TITRE DE L' INVENTION: OLIGONUCLETIDES DERIVES DES GENES VTS DE BACTERIES E. COLI PRODUCTRICES DE VEROTOXINES, ET LEURS UTILISATIONS
   (III) NOMBRE DE SEQUENCES: 8
   (IV) FORME DECHIFFRABLE PAR ORDINATEUR:
      (A) TYPE DE SUPPORT: FLOPPY DISK
      (B) ORDINATEUR: IBM PC COMPATIBLE
      (C) SYSTEME D' EXPLOITATION: PC-DOS/MS-DOS
      (D) LOGICIEL: PATENTIN RELEASE #1.0, VERSION #1.30 (OEB)
(2) INFORMATIONS POUR LA SEQ ID NO: 1:
   (I) CARACTERISTIQUES DE LA SEQUENCE:
      (A) LONGUEUR: 23 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 1:
   ACTGTSACAG CWGAAGCYTT ACG                                   23
(2) INFORMATIONS POUR LA SEQ ID NO: 2:
   (I) CARACTERISTIQUES DE LA SEQUENCE:
      (A) LONGUEUR: 23 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 2:
   SCCACTTTWA CTGTRAAKGT ATC                                   23
(2) INFORMATIONS POUR LA SEQ ID NO: 3:
   (I) CARACTERISTIQUES DE LA SEQUENCE:
      (A) LONGUEUR: 29 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 3:
   GRAGAHGTKG AYCTYACWYT GAACTGGGG 29
(2) INFORMATIONS POUR LA SEQ ID NO: 4:
   (I) CARACTERISTIQUES DE LA SEQUENCE:
      (A) LONGUEUR: 24 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 4:
   TWATACTGAA TTGYCATCAT CAKG 24
(2) INFORMATIONS POUR LA SEQ ID NO: 5:
   (I) CARACTERISTIQUES DE LA SEQUENCE:
      (A) LONGUEUR: 66 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 5:
   ACTGTCACAG CAGAAGCCTT ACGAGCACCT CAAAAACACC ATCATACACT AAATCAGTAA 60
   GTTGGC 66
(2) INFORMATIONS POUR LA SEQ ID NO: 6:
   (I) CARACTERISTIQUES DE LA SEQUENCE:
      (A) LONGUEUR: 63 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 6:
   CCCACTTTTA CTGTGAATGT ATCGGTAAAC CAGCAATAGA CATAAGCGGC TATTTAACGA 60
   CCC 63
(2) INFORMATIONS POUR LA SEQ ID NO: 7:
   (I) CARACTERISTIQUES DE LA SEQUENCE:
      (A) LONGUEUR: 30 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 7:
   TCGCAAGATG TGGCGTGTTA CGGTGAAAAC 30
(2) INFORMATIONS POUR LA SEQ ID NO: 8:
   (I) CARACTERISTIQUES DE LA SEQUENCE:
      (A) LONGUEUR: 30 PAIRES DE BASES
      (B) TYPE: NUCLEOTIDE
      (C) NOMBRE DE BRINS: SIMPLE
      (D) CONFIGURATION: LINEAIRE
   (XI) DESCRIPTION DE LA SEQUENCE: SEQ ID NO: 8:
   CAAGGCGACA AGGTGCTGAT GCCGCTGGCG 30

## Description

La présente invention est relative à des oligonucléotides permettant la détection des *Escherichia coli* producteurs de vérotoxines (VTEC), et en particulier des *Escherichia coli* entérohémorragiques (EHEC).

Les *Escherichia coli* entérohémorragiques sont de plus en plus fréquemment reconnus comme responsables de toxi-infections alimentaires (GANNON et al., 1993, J. Clin Microbiol. 31 : 1268-1274 ; BEGUM D. et al., 1993, J. Clin. Microbiol. 31 : (12) 3153-3156), dont les conséquences chez l'homme peuvent aller de la simple diarrhée jusqu'au syndrome hémolytique urémique (SHU). Le SHU survient principalement chez les jeunes enfants ; il représente environ 9% des infections à VTEC diagnostiquées aux Etats-Unis. Il est par ailleurs la principale cause d'insuffisance rénale aiguë chez l'enfant, et la létalité observée est de l'ordre de 3 à 5/1000.

Ces germes sont également impliqués chez l'animal, dans des infections digestives plus ou moins graves. Les symptômes observés sont des diarrhées et des colites hémorragiques chez les bovins, la dysenterie chez les veaux ou la maladie de l'oedème chez le porc.

Des cytotoxines produites par les EHEC constituent le principal facteur de pathogénicité impliqué dans ces infections. Ces cytotoxines sont dénommées « Shiga like toxins » (SLT) du fait de leur analogie avec la toxine sécrétée par *Shigella dysenteriae* de type 1, ou également vérotoxines (VT), du fait de leur activité toxique sur les cellules Vero. Les bactéries productrices de ces toxines sont dénommées SLTEC ou VTEC.

Pour prévenir les pathologies associées aux EHEC, il serait nécessaire d'être en mesure de contrôler systématiquement les aliments susceptibles d'être contaminés par ces bactéries, c'est à dire en particulier les produits frais.

Initialement, un seul sérotype d'*E. coli* avait été associé au SHU : il s'agit du sérotype Ol57:H7, qui est celui le plus fréquemment rencontré aux Etats-Unis et au Canada. Il a été proposé de détecter ce sérotype à l'aide de techniques biochimiques basées sur la fermentation du sorbitol et la production de béta-glucuronidase.

Toutefois, il a ultérieurement été observé (BRIAN. et al., J. Clin. Microbiol., 30 : (7), 1801-1806, 1992] qu'un grand nombre d'autres sérotypes (O26:H11, O111:H-, O103:H2 pour ne citer que les principaux) pouvaient être impliqués dans les infections digestives à VTEC, et que la détermination du seul sérotype ne constituait pas un bon marqueur de pathogénicité. De plus, si la grande majorité des *E. coli* O157:H7 ne fermentent pas le sorbitol en 24 heures et apparaissent sous forme de colonies blanches sur les géloses à base de sorbitol, il existe un certain nombre de mutants qui sont capables de fermenter le sorbitol. Les techniques biochimiques sont donc devenues insuffisantes car elles se limitent à l'identification du seul sérotype Ol57:H7 en général sorbitol négatif, alors que la majorité des EHEC non-0157 sont capables de -fermenter le sorbitol et apparaissent sous forme de colonies pigmentées, impossibles à discriminer des nombreux colibacilles non-pathogènes.

Plusieurs types antigéniques de vérotoxines ont été décrits. Les types principaux sont les vérotoxines VT1 (SLT 1), VT2 (SLT2) ; on distingue aussi des variants comme VT2 vha, VT2 vhb, VTE (SLT II-v) et VTE-va (SLT II-va) [ZAW LIN et al., Microbiol. Immunol., 37 : :(7) 543-548 (1993)].

Il a été proposé de détecter ces vérotoxines â l'aide d'anticorps spécifiques, par des techniques immunologiques de type ELISA. Cependant, celles-ci sont en général relativement complexes à mettre en oeuvre ou trop peu sensibles pour permettre une détection fiable dans les aliments. En outre, la plupart des tests immunologiques actuellement disponibles sont limités à la détection de Ol57:H7 et ne permettent pas la recherche de l'ensemble des sérotypes producteurs de vérotoxines.

La méthode de référence utilisée en hygiène alimentaire pour détecter la présence de bactéries productrices de vérotoxines, repose sur des tests de cytotoxicité sur des cultures de cellules. Le principe de ces tests est basé sur la cytopathogénicité des vérotoxines sur des cellules de rein de singe vert d'Afrique. Cette technique a l'avantage d'être très sensible ; cependant il faut compter 5 à 6 jours avant d'obtenir les premiers résultats et les coûts sont élevés. Une série de transferts en milieux sélectifs est nécessaire afin de favoriser le développement des bactéries vérotoxiques préalablement au dosage des toxines sur une culture de cellules. Par la suite, une confirmation des types antigéniques de vérotoxine est nécessaire à l'aide d'anticorps anti-VT1 et anti-VT2. Ce type de test ne constitue donc pas une méthode de routine utilisable dans l'industrie agro-alimentaire.

Il a également été proposé de détecter les VTEC à l'aide d'oligonucléotides (sondes nucléiques ou amorces d'amplification génique) spécifiques des gènes (dénommés gènes *vts* ou gènes SLT) codant pour les vérotoxines.

Pour être en mesure de détecter différentes souches de VTEC, les techniques d'amplification génique généralement proposées font appel soit à des cocktails d'amorces (PCR multiplex) permettant de détecter simultanément les différents gènes des vérotoxines, soit à un couple unique d'amorces permettant d'amplifier une région conservée de ces gènes. KARCH et MEYER [J. Clin. Microbiol., 27, 2751-2757, (1989)] ont ainsi décrit des amorces, déduites d'une région conservée des gènes SLT, et permettant d'amplifier un segment de SLT1 ou de SLT2 ; READ et al., [Mol. and Cell. Probes, 6,153-161 (1992)] ont également décrit des amorces permettant d'amplifier une région conservée des gènes VT1, VT2, et VTE ; ces amorces amplifient un fragment de 323 pb chez les VTEC, et chez *Shigella dysenteriae*, et des fragments de 900pb et 1200pb, respectivement, chez 2 *Salmonella*, et chez *Proteus mirabilis ;* ce test permet de détecter environ 10² bactéries, à partir de souches pures. ZAW LIN et al. [Microbiol. Immunol., 37, 543-548 (1993)] ont également développé des amorces de PCR qui amplifient une région conservée d'environ 900 pb des gènes *vts ;* ils indiquent qu'aucun produit d'amplification de cette taille n'est obtenu avec des souches non-VTEC ; ils ne précisent pas le seuil de sensibilité de leur méthode. PATON et al., [J. Clin. Microbiol., 3063-3067 (1993)] décrivent des amorces qui amplifient une région conservée de 215 pb environ des gènes SLT1 et SLT2 ; le seuil de détection est d'environ 10 bactéries/ml ; des produits d'amplification de taille non-spécifique sont observés avec une souche témoin d*'E*.*coli*.

Les Inventeurs ont maintenant sélectionné des régions très conservées des gènes *vts* des vérotoxines, différentes de celles décrites dans les publications citées ci-dessus, et sont parvenus à obtenir des préparations d'oligonucléotides dégénérés capables de détecter l'ensemble des gènes *vts* codant pour les principaux types antigéniques de vérotoxines (VT1, VT2, VTE) dans les échantillons biologiques, sans présenter d'hybridation croisée avec des régions du génome d'autres espèces bactériennes.

La présente invention a pour objet un oligonucléotide ou un mélange d'oligonucléotides choisis parmi :
- un oligonucléotide, ou un mélange d'oligonucléotides identiques ou différents, définis par la séquence générale EH1 suivante :
- un oligonucléotide, ou un mélange d'oligonucléotides identiques ou différents, définis par la séquence générale EH2 suivante :
- un oligonucléotide, ou un mélange d'oligonucléotides identiques ou différents, définis par la séquence générale EH3 suivante :
dans lesquelles S représente G ou C, W représente A ou T, Y représente T ou C, R représente A ou G, K représente G ou T, H représente A, C, ou T.

Les séquences des oligonucléotides EH1, EH2, EH3 et EH4 sont représentées dans la liste de séquences en annexe sous les numéros respectifs SEQ ID NO: 1, SEQ ID NO: 2, SEQ ID NO: 3, et SEQ ID NO: 4.

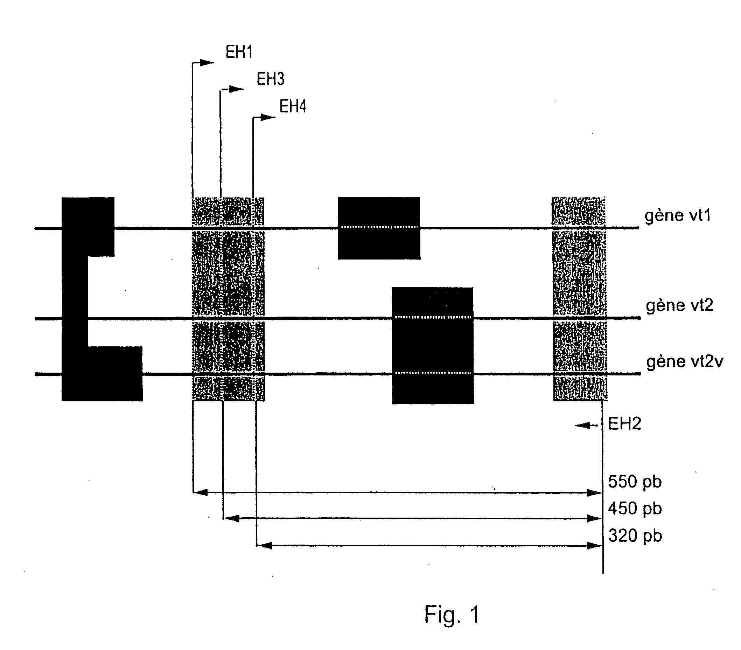

L'emplacement des oligonucléotides EH1, EH2, EH3 et EH4 par rapport aux gènes *vts* est représenté sur la figure 1
■ = régions conservées ;
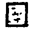 = régions hyperconservées.

Ces oligonucléotides permettent de constituer des sondes utiles pour détecter les gènes *vts*, ou des couples d'amorces utilisables dans une technique d'amplification génique, par exemple dans l'amplification en chaîne par polymérase (PCR).

La présente invention a également pour objet les couples d'amorces constitués par l'association de l'oligonucléotide EH2 avec l'un des oligonucléotides EH1, EH3, ou avec un oligonucléotide, ou un mélange d'oligonucléotides identiques ou différents, définis par la séquence générale EH4 suivante : dans laquelle S, W, Y, R, K et H sont tels que définis ci-dessus.

Les couples d'amorces EH1/EH2, EH3/EH2 et EH4/EH2 amplifient respectivement des fragments de 550 pb, 450 pb et 320 pb.

Les séquences des oligonucléotides EH3 et EH4 sont situées à l'intérieur du fragment de 550 pb délimité par EH1/EH2. EH3 et EH4 peuvent par conséquent être utilisées comme sondes internes pour identifier le fragment de 550 pb. De même, EH4 peut être utilisé pour identifier le fragment de 450 pb délimité par EH3/EH2. Ces oligonucléotides permettent de détecter les gènes VT1, VT2, et VTE codant pour les différents types de toxines.

Ces couples d'amorces peuvent également être utilisés pour effectuer une bi-amplification (hémi-nested PCR). Par exemple, le produit d'amplification obtenu avec EH1/EH2 peut être utilisé comme matrice pour une nouvelle amplification avec EH3/EH2 ou EH4/EH2, et celui obtenu avec EH3/EH2 peut être utilisé comme matrice pour une nouvelle amplification avec EH4/EH2.

Ils peuvent aussi être utilisés dans une technique du type « PCR-ELISA ». Dans ce cas, on peut, par exemple, utiliser l'oligonucléotide EH3 préalablement immobilisé sur un support solide, en tant que sonde de capture pour fixer le produit d'amplification de 550 bp obtenu avec les amorces EH1 et EH2 ; ce produit d'amplification peut alors être révélé soit directement (par exemple s'il a été marqué de façon appropriée par incorporation de nucléotides marqués au cours de la réaction d'amplification) soit par l'intermédiaire d'une sonde de détection marquée (par exemple l'oligonucléotide EH4). Alternativement, EH4 peut être utilisé en tant que sonde de capture, et EH3 en tant que sonde de détection.

Différentes méthodes pour fixer des acides nucléiques sur support solide, ainsi que celles pour les marquer à l'aide de marqueurs froids ou radioactifs sont bien connues de l'homme du métier, à titre d'exemple on citera celles décrites par SAMBROOK et al., dans : Molecular Cloning : A Laboratory Manual, Cold Spring Harbor Laboratory Press, Cold Spring Harbor, N.Y. (1989).

Les oligonucléotides conformes à l'invention permettent donc la mise en oeuvre de méthodes de détection de VTEC qui sont simples à mettre en oeuvre, faciles à automatiser, et utilisables en particulier dans le cadre d'un diagnostic de masse.

Avantageusement, pour la mise en oeuvre de ces méthodes de détection de VTEC dans un échantillon biologique, on utilise un contrôle interne d'amplification, dans le but d'évaluer une éventuelle inhibition de la PCR due à des substances présentes dans les échantillons biologiques analysés (par exemple produits alimentaires ou échantillons de matières fécales).

Ce contrôle interne est constitué par une molécule d'acide nucléique, par exemple un plasmide, comprenant une séquence déterminée pouvant être amplifiée en utilisant l'un des couples d'amorces conformes à l'invention. Cette séquence est constituée par une séquence interne X, n'ayant pas de séquences communes avec les fragments de 550 pb, 450 pb ou 320 pb, définis ci-dessus, des gènes vts, flanquée à une extrémité d'une séquence constituée par l'oligonucléotide EH2 et son brin complémentaire, et à l'autre extrémité d'une séquence constituée soit par l'oligonucléotide EH1 et son brin complémentaire, soit par l'oligonucléotide EH3 et son brin complémentaire, soit par l'oligonucléotide EH4 et son brin complémentaire. Le produit d'amplification de cette séquence peut être détecté à l'aide de sondes spécifiques de la séquence interne X. Avantageusement, l'ensemble est inséré dans un plasmide, qui peut être préparé et purifié commodément en quantités importantes.

Le plasmide ainsi préparé est ajouté à l'échantillon biologique à analyser, et l'on procède à l'amplification avec un couple d'amorces conformes à l'invention (par exemple EH1 et EH2), et à la détection des produits issus de l'amplification des gènes vts (par exemple en utilisant EH3 et/ou EH4 en tant que sondes internes) et des produits issus de l'amplification de la séquence contrôle, à l'aide des sondes spécifiques de la séquence interne X.

Il est également avantageux d'utiliser en outre un témoin positif à titre de contrôle interne, en particulier pour vérifier la sensibilité de la détection ; ce contrôle positif est constitué par l'un des fragments de 550 pb, 450 pb ou 320 pb, définis ci-dessus, des gènes *vts* ; il peut donc être amplifié, et son produit d'amplification détecté, en utilisant les oligonucléotides conformes à l'invention.

Les Inventeurs ont comparé les résultats obtenus en mettant en oeuvre un procédé d'amplification génique utilisant des oligonucléotides conformes à l'invention avec ceux obtenus en mettant en oeuvre la méthode de référence d'essais de cytotoxicité sur cellules Vero. Cette comparaison montre que la détection de VTEC à l'aide d'oligonucléotides conformes à l'invention, est au moins aussi sensible que la méthode de référence.

Les oligonucléotides conformes à l'invention permettent de détecter toutes les bactéries VTEC. Si l'on souhaite déterminer plus précisément le type toxinique, on peut éventuellement mettre en oeuvre une étape de détection utilisant les oligonucléotides conformes à l'invention, et une étape de détection utilisant des amorces plus spécifiques d'un gène *vts* déterminé.

Les oligonucléotides conformes à l'invention sont utilisables pour le dépistage de bactéries VTEC dans les produits alimentaires, en particulier les produits alimentaires frais tels que les viandes ou les produits dérivés, ou bien les produits laitiers.

Ils peuvent également être utilisés pour le diagnostic des infections à VTEC des humains et des animaux, à partir d'un échantillon biologique, par exemple dans les selles.

L'amplification génique effectuée en utilisant comme amorces les oligonucléotides conformes à l'invention permet d'abaisser le seuil de détection dans les aliments, jusqu'à environ 10 VTEC/25 g.

La présente invention sera mieux comprise à l'aide du complément de description qui va suivre, qui se réfère à des exemples non-limitatifs illustrant la mise en oeuvre d'oligonucléotides conformes à l'invention pour la détection de VTEC.

### Exemple 1 : étude de la spécificité des oligonucléotides EH1, EH2, EH3, et EH4

### 1) Extraction de l'ADN bactérien :

Les souches bactériennes sont cultivées sur milieu TSB (Trypticase Soja Broth), à 37°C, pendant 5 heures environ, puis les cultures sont centrifugées à 12000 rpm, pendant 2 minutes à 4°C. Le culot bactérien est rincé à l'eau distillée stérile, puis récupéré par centrifugation (2 min. à 12000 rpm, à 4°C). 200 µl de CHELEX® 100 (BIO RAD) sont ajoutés au culot. Ce mélange est agité, puis mis à incuber 30 min. à 56°C, puis 8 à 10 min. à 100°C, et enfin centrifugé 1 à 2 min. à 12000 rpm, à 4°C. L'ADN purifié se retrouve alors dans le surnageant.

### 2)Amplification des séquences des gènes vts

Des préparations d'oligonucléotides dégénérés EH1, EH2, EH3, et EH4 sont obtenus par des techniques classiques de synthèse oligonucléotidique.

Les conditions d'amplification utilisées sont les suivantes : l'ADN extrait (2-3 µl) est mis en présence de désoxyribonucléotides (200 µM), de tampon Taq polymérase (BOEHRINGER) x 1, de 0,4 µM de chaque amorce, et de Taq polymérase (2,5 Unités). La réaction s'effectue sous un volume final de 50 µl.

On effectue 45 cycles alternant une étape de fixation des amorces à 58°C pendant 30 secondes, une étape d'extension à 72°C pendant 30 secondes, et une étape de dénaturation à 95°C pendant 30 secondes, et on termine par une étape d'extension à 72°C pendant 10 minutes.

Pour déterminer la spécificité des couples oligonucléotides EH1/EH2, EH2/EH3 et EH3/EH4, l'amplification a été effectuée à partir d'ADN extrait de :
- 24 souches de VTEC regroupant 11 sérotypes, dont le sérotype 0157:H7.
- 66 souches bactériennes non-VTEC comprenant des *E. coli* non vérotoxiques et des bactéries proches de *E. Coli,* ou des bactéries pathogènes ou non, pouvant être rencontrées dans les aliments, et appartenant en particulier aux genres *Lactobacillus, Listeria, Staphylococcus, Salmonella, Proteus, Shigella.*

Pour chacune des 24 souches d'*E. coli* productrices de vérotoxines testées avec le couple d'amorces EH1/EH2 un fragment de 550 pb a pu être observé après électrophorèse sur gel d'agarose. Toutes ces souches produisent également un fragment d'amplification de 450 pb avec le couple d'amorces EH2/EH3 et un fragment d'amplification de 320 pb avec le couple EH2/EH4.

En revanche, l'ADN extrait des 66 espèces bactériennes non productrices de vérotoxines n'a donné, dans les mêmes conditions et avec les mêmes couples d'amorces, aucun fragment d'amplification.

Ces résultats montrent que les oligonucléotides EH1, EH2, EH3 et EH4 sont spécifiques des gênes *vts.* Ils peuvent donc être utilisés pour identifier spécifiquement les *E. coli* vérotoxiques, par la présence de fragments d'amplification.

### Exemple 2 : Détermination de la sensibilité du test sur des souches pures de VTEC.

La sensibilité du test a été évaluée sur de l'ADN de 3 souches VTEC de référence : ATCC H19, ATCC EDL 933 et ATCC B2F1, en déterminant le seuil limite de détection du test après amplification simple, ou avec différents couples d'amorces, en utilisant le protocole décrit dans l'exemple 1 ci-dessus.

L'amplification simple a été effectuée avec le couple d'amorces EH1/EH2, ou bien avec le couple d'amorces EH2/EH3.

Dans le cas de la bi-amplification, ou bien une première amplification réalisée avec le couple d'amorces EH1/EH2 a été suivie d'une seconde amplification avec le couple d'amorces internes EH2/EH3, ou bien une première amplification réalisée avec le couple d'amorces EH2/EH3 a été suivie d'une seconde amplification avec le couple d'amorces internes EH2/EH4 (hémi-nested PCR).

Les résultats sont illustrés par le tableau 1 ci-dessous. Ces résultats montrent qu'après une simple amplification, le seuil de détection est de 5 à 100 bactéries dans la prise d'essai lorsqu'on utilise le couple EH1/EH2 seul. Avec le couple EH2/EH3 seul, le seuil de détection est plus faible : 0,01 à 5 bactéries détectées après simple amplification. Dans ce dernier cas, la bi-amplification avec EH2/EH4 n'abaisse pas le seuil de détection qui reste compris entre 0,01 et 5 bactéries. En revanche, lorsque les produits d'amplification obtenus avec EH1/EH2 sont ré-amplifiés avec EH2/EH3, la bi-amplification, obtenue avec EH2/EH3 (hémi-nested PCR) permet d'abaisser le seuil de détection entre 0,01 et 50 bactéries en fonction des souches testées.

**TABLEAU I**

| SOUCHE | SEROTYPE | SLT | EH1/EH2 | EH1/EH2 + EH2/EH3 | EH2/EH3 | EH2/EH3 + EH3/EH4 |
|---|---|---|---|---|---|---|
| ATCC H19 | O26:H11 | VT1 | 5<X<50 | 5<X<50 | 0,5<X<5 | 0,5<X<5 |
| ATCC EDL933 | O157:H7 | VT1/VT2 | 10<X<100 | 10⁻²<X<10⁻¹ | 10⁻²<X<10⁻¹ | 10⁻²<X<10⁻¹ |
| ATCC B2F1 | O91:H21 | VT2V | 1<X<10 | 10⁻¹<X<1 | 10⁻¹<X<1 | 10⁻¹<X<1 |
| X : nombre de bactéries détectées dans la prise d'essai. | | | | | | |

### Exemple 3 : Détection de VTEC dans des produits alimentaires artificiellement contaminés

### 1) Détection par amplification génique :

45 échantillons de fromages au lait cru (camembert, cantal, Pont l'Evêque, reblochon) ont été contaminés par des souches de VTEC, selon 3 taux théoriques de contamination initiale: 10³, 10² et 10 bactéries par 25 g. d'aliment. Les souches de VTEC utilisées pour la contamination sont d'origine alimentaire et humaine (souches SA1, SA2, SA5, SA6, SA7 et SA8).

Les enrichissements ont été réalisés selon le protocole préconisé par le Laboratoire d'Expertises et d'Analyses Alimentaires du Québec (LEAA) [P. CARDINAL : « Les *E. coli* producteurs de vérotoxines (*E. coli* 0157:H7) ; résumé des activités de la direction générale de la qualité des aliments et de la santé animale (1987-1993) » (1993) ].

Au stade "BHI-24 heures" 1,5 ml des suspensions alimentaires enrichies sont prélevés en évitant les souillures alimentaires, et centrifugés 1 min. à 700 rpm, à 4°C. Le surnageant est alors centrifugé 2 min., à 12000 rpm, à 4°C. L'extraction d'ADN et l'amplification génique sont réalisées selon les protocoles décrits à l'exemple 1.

La première amplification a été réalisée avec le couple d'amorces EH1/EH2 et la bi-amplification avec le couple d'amorces EH2/EH3.

### 2) Détection de l'effet cytotoxique :

Parallèlement, on a effectué, à titre de comparaison, une recherche des vérotoxines par le test de cytotoxicité sur cellules.

Les cellules Véro sont cultivées à 37°C et en présence de C02 (5%)en milieu de base MEM, supplémenté en ampicilline, en glutamine, en gentamycine et en HEPES. Après rinçage (en milieu MEM supplémenté), les cellules sont soumises à l'action de 2 ml de trypsine 0,25% (dans du PBS), pour les détacher des parois du flacon de culture. Cette première solution de trypsine est ensuite éliminée, puis 3 ml de trypsine 0,25% sont à nouveau ajoutés (30 sec.) aux cellules.

Après élimination de cette deuxième solution, les cellules sont incubées 1 à 2 min. à 37°C.

Lorsque toutes les cellules apparaissent rondes et non-confluentes au microscope inversé, elles sont reprises dans 5 ml de milieu nutritif (milieu de base supplémenté en sérum de veau foetal 10%). Une centrifugation de 15 min. à 1000 rpm permet de collecter les cellules, qui sont ensuite reprises en milieu de base supplémenté en HEPES 25 mM (2 x 10⁵ cellules/ml). Cette solution est ensuite répartie dans les plaques de culture (200µl/puits) qui sont alors incubées à 37°C jusqu'à confluence des cellules (2 jours environ).

1 ml de suspension alimentaire enrichie (stade BHI-24 h.) est traité au sulfate de polymyxine B selon le protocole décrit par EVANS et al. [EVANS et al., Infect. Immun. 10, 1010-1017 (1974)] pour libérer les cytotoxines Shiga like.

Pour réaliser le test de cytotoxicité, 100µl de solutions contenant les toxines sont diluées au 1/10 puis 7 fois au 1/2 en milieu de base. Chaque dilution est ensuite ajoutée aux cellules Véro confluentes (1:1). La plaque de culture est alors incubée jusqu'à 72 h. à 37°C. L'activité cytotoxique est surveillée au microscope inversé (x 200) dès 24 h. d'incubation.

Après 72 h. d'incubation, les cellules sont fixées 1 min. avec une solution de formaldéhyde (2%) PBS (0,067 M, pH 7,2).

La coloration des cellules (10 min. dans une solution de coloration violet cristal 0,13%, éthanol 5%, formaldéhyde 2%, PBS) permet de différencier les cellules mortes des cellules vivantes : seules les cellules vivantes prennent une coloration violette.

La détermination du type toxinique est réalisée par séroneutralisation de l'effet cytotoxique à l'aide d'anticorps anti-VT. Un sérum anti-VT1 (titre :1/1024) est dilué (1/500) en milieu de base. Les surnageants de culture sont dilués 8 fois successivement (1/10), puis chaque dilution est mélangée au sérum dilué (1:1). 100 µl de ce mélange sont ajoutés dans les micropuits contenant les cellules Véro confluentes. Le suivi de l'activité cytotoxique est similaire à celui décrit ci-dessus.

### 3) Résultats :

Que ce soit avec le test d'amplification génique utilisant les oligonucléotides conformes à l'invention, ou avec le test de culture cellulaire de référence, il a été possible de détecter jusqu'à 10 VTEC/25 g. d'aliment.

Les résultats respectivement obtenus avec le test (BM) et le test de culture cellulaire (CC) sont comparés dans le tableau II ci dessous.

**TABLEAU II.**

| ALIMENTS | N | BM+/CC+ | BM-/CC- | BM+/CC- | BM-/CC+ |
|---|---|---|---|---|---|
| Camembert | 24 | 15 | 7 | 2 | O |
| Cantal | 12 | 9 | 3 | O | O |
| Pont l'Evêque | 12 | 3 | 6 | 2 | 1 |
| Reblochon | 12 | 4 | 7 | 1 | O |
| TOTAL | 60 | 31 | 23 | 5 | 1 |
| N : nombre d'analyses réalisées | | | | | |
| BM : test d'amplification génique | | | | | |
| CC : test de culture cellulaire | | | | | |

Tous les échantillons positifs avec le test de culture cellulaire sont également positifs avec le test d'amplification génique (31 échantillons), excepté 1 échantillon (Pont l'Evêque) pour lequel le test d'amplification génique est resté négatif. On note cependant que le test d'amplification génique utilisant les oligonucléotides conformes à l'invention s'avère légèrement plus sensible que le test de référence : 36 échantillons détectés à l'aide des oligonucléotides conformes à l'invention, contre 32 échantillons avec le test de référence.

### Exemple 4 : Préparation d'un plasmide de contrôle interne utilisable pour évaluer une inhibition éventuelle de la réaction d'amplification

Pour des facilités de clonage et de sélection du clone recombinant, un gène de résistance au chloramphénicol *(Cm)* du Tn9 porté par le plasmide PACYC 184 commercialisé par BIOLABS (Genbank accession: 06403) a été choisi comme insert.

Le couple d'amorces (C11 et C12) qui a servi à l'amplification du gène *(Cm)* a été choisi pour insérer de part et d'autre du gène *(Cm)* les séquences des oligonucléotides EH1 et EH2.

L'amorce C11 est ainsi constituée de l'amorce EH1 en position 5' et des bases complémentaires de la région 494-452 du PACYC 184 en position 3' ; sa séquence est la suivante :
Oligonucléotide C11 :

La séquence de cet oligonucléotide C11 est représentée dans la liste de séquences en annexe sous le numéro SEQ ID NO: 5.

L'amorce C12 est ainsi constituée de l'amorce EH2 en position 5' et des bases de la région 3645-3684 du PACYC184 en position 3' ; sa séquence est la suivante :
Oligonucléotide C12 :

La séquence de cet oligonucléotide C12 est représentée dans la liste de séquences en annexe sous le numéro SEQ ID NO: 6.

Après 35 cycles d'amplification avec une température d'hybridation de 57°C, un produit d'amplification contenant le gène Cm de 1141 bp a été obtenu et purifié par phénol/chloroforme, et précipitation à l'éthanol.

Le produit d'amplification a été cloné au niveau du site *EcoRV* du plasmide pMOSBlue T-vector® portant un marqueur de résistance à l'ampicilline, à l'aide du pMOSBlue T-vector kit (AMERSHAM).

Après ligature et transformation de *E. coli* MOS *Blue*, le clone T3 portant le vecteur recombinant résultant a été sélectionné sur milieu gélosé contenant de l'ampicilline et du chloramphénicol. (Le clone T3 recombinant exprime la résistance au chloramphénicol).

Le plasmide recombinant de 4028pb (= contrôle interne T3) a été purifié à partir de 100 ml de culture. Plus de 100 µg de plasmide ont été obtenus. Ce plasmide est reconnu et amplifié avec le couple d'amorces EH1 et EH2. Il produit un fragment d'amplification de 1141 bp, dans les mêmes conditions d'amplification génique que celles décrites ci-dessus pour l'obtention du fragment de 550 bp chez les VTEC. Il peut par conséquent être utilisé comme contrôle interne d'une éventuelle inhibition dans les amplifications par EH1 et EH2.

Pour permettre la détection par hybridation ADN/ADN des produits de 1141 bp générés par l'amplification par EH1 et EH2 du contrôle interne, deux oligonucléotides nommés CATcap et CATrev ont été choisis à l'intérieur de la séquence du gène *Cm*.

Ces oligonucléotides peuvent être utilisés indifféremment comme sonde de capture et sonde de révélation dans une réaction d'hybridation sandwich pour détecter le produit d'amplification de 1141 bp correspondant au contrôle interne T3.

L'oligonucléotide CATcap correspond aux bases complémentaires des positions 4104-4075 du plasmide PACYC184. Sa séquence est la suivante :

La séquence de cet oligonucléotide CATcap est représentée dans la liste de séquences en annexe sous le numéro SEQ ID NO: 7.

CATcap marqué en 5' à la biotine a servi à la capture du produit d'amplification de 1141 bp sur des microplaques sensibilisées à la streptavidine.

L'oligonucléotide CATrev correspond aux bases complémentaires des positions 3930-3901 du plasmide PACYC184. Sa séquence est la suivante :

La séquence de cet oligonucléotide CATrev est représentée dans la liste de séquences en annexe sous le numéro SEQ ID NO: 8.

CATrev marqué en 3' à la digoxigénine a permis la révélation par chemioluminescence du produit d'amplification de 1141 bp.

Les sondes CATcap et CATrev sont spécifiques du fragment de 1141 bp ; elles ne réagissent pas de façon croisée avec le produit d'amplification de 550 bp obtenu à partir des gènes VTs.

La co-amplification par EH1 et EH2 de souches de VTEC ou d'échantillons biologiques contaminés par VTEC et du contrôle interne, suivie de la double détection des produits issus de l'amplification des gènes de vérotoxines par EH3 et EH4 et des produits issus de l'amplification du contrôle interne par CATrev et CATcap a permis de vérifier que le plasmide recombinant T3 est un bon indicateur d'une éventuelle inhibition de la réaction d'amplification.

### Exemple 5 : Préparation d'un plasmide de contrôle interne utilisable comme témoin positif d'amplification.

Pour obtenir un contrôle positif, un plasmide recombinant purifié, ayant comme insert le fragment d'amplification de 550 bp obtenu avec les amorces EH1 et EH2 a été préparé.

Un produit d'amplification de 550 bp a été obtenu à partir de la souche E32511 de *E. coli* Ol57:H7 à l'aide des amorces EH1 et EH2, comme décrit à l'exemple 1 ci-dessus.

Le produit d'amplification a été cloné au niveau du site EcoRV du pMOSBlue® T-vector (portant la résistance à l'ampicilline) à l'aide du *pMOSBlue*® T-vector kit (AMERSHAM). Après ligature et transformation de *E. coli* MOS *Blue,* le clone T2 portant le vecteur recombinant résultant a été sélectionné sur milieu gélosé contenant de l'ampicilline.

Le plasmide recombinant de 3437bp (= contrôle positif T2) a été purifié à partir de 100 ml de culture. Plus de 100 µg de plasmide ont été obtenus. Ce plasmide est reconnu et amplifié avec le couple d'amorces EH1 et EH2. Il produit un fragment d'amplification de 550 bp caractéristique des VTEC qui peut s'hybrider avec les amorces EH3 et EH4. Le plasmide recombinant T2 peut par conséquent être utilisé comme contrôle positif dans les amplifications par EH1 et EH2.

## Revendications

1. Oligonucléotide ou mélange d'oligonucléotides utilisables comme amorces pour la détection des bactéries vérotoxiques par amplification génique des gènes vts, **caractérisés en ce qu'**ils sont choisis parmi :
- un oligonucléotide, ou un mélange d'oligonucléotides identiques ou différents, définis par la séquence générale EH1 (SEQ ID NO: 1) suivante :
- un oligonucléotide, ou un mélange d'oligonucléotides identiques ou différents, définis par la séquence générale EH2 (SEQ ID NO: 2) suivante :
- un oligonucléotide, ou un mélange d'oligonucléotides identiques ou différents, définis par la séquence générale EH3 (SEQ ID NO: 3) suivante : dans lesquelles S représente G ou C, W représente A ou T, Y représente T ou C, R représente A ou G, K représente G ou T, H représente A, C, ou T.

2. Couple d'amorces pour l'amplification génique des gènes vts, **caractérisé en ce qu'**il est constitué par un oligonucléotide ou un mélange d'oligonucléotides de séquence générale SEQ ID NO: 2 (EH2) avec un oligonucléotide, ou un mélange d'oligonucléotides choisis parmi :
- un oligonucléotide, ou un mélange d'oligonucléotides de séquence générale SEQ ID NO: 1 (EH1);
- un oligonucléotide, ou un mélange d'oligonucléotides de séquence générale SEQ ID NO: 3 (EH3);
- un oligonucléotide, ou un mélange d'oligonucléotides identiques ou différents, définis par la séquence générale EH4 (SEQ ID NO: 4) suivante : dans laquelle S représente G ou C, W représente A ou T, Y représente T ou C, R représente A ou G, K représente G ou T, H représente A, C, ou T.

3. Procédé d'amplification génique d'une portion d'ADN commune aux gènes VT1, VT2 et VTE des bactéries vérotoxiques, **caractérisé en ce que** l'on utilise comme amorces :
- un oligonucléotide ou un mélange d'oligonucléotides définis par la séquence générale EH2 (SEQ ID NO: 2) et,
- un oligonucléotide ou un mélange d'oligonucléotides définis par l'une des séquences générales EH1 (SEQ ID NO: 1), EH3 (SEQ ID NO: 3), ou EH4 (SEQ ID NO: 4).

4. Procédé selon la revendication 3 **caractérisé en ce qu'**il comprend une première amplification au cours de laquelle on utilise comme amorces les oligonucléotides ou mélanges d'oligonucléotides définis par les séquences générales EH1 et EH2, et une seconde amplification, au cours de laquelle l'on utilise comme matrice le produit d'amplification de la première étape, et comme amorces les oligonucléotides ou mélanges d'oligonucléotides définis par les séquences générales EH2 et EH3 ou EH2 et EH4.

5. Procédé selon la revendication 3, **caractérisé en ce qu'**il comprend une étape d'amplification au cours de laquelle on utilise comme amorces les oligonucléotides ou mélanges d'oligonucléotides définis par les séquences générales EH1 et EH2, et une étape de capture du produit d'amplification sur un support solide, dans laquelle on utilise comme sonde de capture au moins un des oligonucléotides ou mélanges d'oligonucléotides définis par les séquences générales EH3 et EH4, préalablement fixé audit support solide.

6. Procédé selon la revendication 3, **caractérisé en ce qu'**il comprend une étape d'amplification au cours de laquelle on utilise comme amorces les oligonucléotides ou mélanges d'oligonucléotides définis par les séquences générales EH1 et EH2, et une étape de détection du produit d'amplification, dans laquelle on utilise comme sonde au moins un des oligonucléotides ou mélanges d'oligonucléotides définis par les séquences générales EH3 et EH4.

7. Procédé selon la revendication 3, **caractérisé en ce que** :
- on utilise comme amorces un oligonucléotide ou un mélange d'oligonucléotides définis par la séquence générale EH2 et un oligonucléotide ou un mélange d'oligonucléotides définis par la séquence générale EH1, pour obtenir un fragment d'amplification de 550 pb ; ou
- on utilise comme amorces un oligonucléotide ou un mélange d'oligonucléotides définis par la séquence générale EH2 et un oligonucléotide ou un mélange d'oligonucléotides définis par la séquence générale EH3, pour obtenir un fragment d'amplification de 450 pb ; ou
- on utilise comme amorces un oligonucléotide ou un mélange d'oligonucléotides définis par la séquence générale EH2 et un oligonucléotide ou un mélange d'oligonucléotides définis par la séquence générale EH4, pour obtenir un fragment d'amplification de 320 pb.

8. Procédé de préparation d'un plasmide **caractérisé en ce qu'**il comprend:
- la mise en oeuvre d'un procédé selon la revendication 7,
- l'insertion du fragment d'amplification obtenu dans un plasmide hôte.

9. Utilisation d'au moins un oligonucléotide, ou un mélange d'oligonucléotides selon la revendication 1, ou d'un couple d'amorces selon la revendication 2, pour la détection de souches d'*E. coli* productrices de vérotoxines.

10. Utilisation d'au moins un oligonucléotide, ou un mélange d'oligonucléotides selon la revendication 1, ou d'un couple d'amorces selon la revendication 2, pour la recherche de souches d'*E*. *coli* productrices de vérotoxines, dans des produits alimentaires.

11. Utilisation d'au moins un oligonucléotide, ou un mélange d'oligonucléotides selon la revendication 1, ou d'un couple d'amorces selon la revendication 2 pour la recherche de souches d'*E. coli* productrices de vérotoxines dans un échantillon de fèces.

## Patentansprüche

1. Oligonukleotid oder Oligonukleotidmischung, die sich als Primer zum Nachweis von verotoxischen Bakterien mittels Genamplifikation der vts-Gene eignen, **dadurch gekennzeichnet, daß** sie ausgewählt sind aus:
- einem Oligonukleotid oder einer Mischung von Oligonukleotiden, die gleich oder verschieden sind, definiert durch die folgende allgemeine Sequenz EH1 (SEQ ID NO: 1):
- einem Oligonukleotid oder einer Mischung von Oligonukleotiden, die gleich oder verschieden sind, definiert durch die folgende allgemeine Sequenz EH2 (SEQ ID NO: 2):
- einem Oligonukleotid oder einer Mischung von Oligonukleotiden, die gleich oder verschieden sind, definiert durch die folgende allgemeine Sequenz EH3 (SEQ ID NO: 3):
worin S G oder C bedeutet, W A oder T bedeutet, Y T oder C bedeutet, R A oder G bedeutet, K G oder T bedeutet, H A, C oder T bedeutet.

2. Primerpaar zur Genamplifikation der vts-Gene, **dadurch gekennzeichnet, daß** es aus einem Oligonukleotid oder einer Mischung von Oligonukleotiden der allgemeinen Sequenz SEQ ID NO: 2 (EH2) und einem Oligonukleotid oder einer Mischung von Oligonukleotiden besteht, die ausgewählt sind aus:
- einem Oligonukleotid oder einer Mischung von Oligonukleotiden der allgemeinen Sequenz SEQ ID NO: 1 (EH1);
- einem Oligonukleotid oder einer Mischung von Oligonukleotiden der allgemeinen Sequenz SEQ ID NO: 3 (EH3);
- einem Oligonukleotid oder einer Mischung von Oligonukleotiden, die gleich oder verschieden sind, definiert durch die folgende allgemeine Sequenz EH4 (SEQ ID NO: 4):
worin S G oder C bedeutet, W A oder T bedeutet, Y T oder C bedeutet, R A oder G bedeutet, K G oder T bedeutet, H A, C oder T bedeutet.

3. Verfahren zur Genamplifikation eines DNA-Stücks, das den Genen VT1, VT2 und VTE der verotoxischen Bakterien gemeinsam ist, **dadurch gekennzeichnet, daß** man als Primer einsetzt:
- ein Oligonukleotid oder eine Mischung von Oligonukleotiden, definiert durch die allgemeine Sequenz EH2 (SEQ ID NO: 2), und
- ein Oligonukleotid oder eine Mischung von Oligonukleotiden, definiert durch eine der allgemeinen Sequenzen EH1 (SEQ ID NO: 1), EH3 (SEQ ID NO: 3) oder EH4 (SEQ ID NO: 4).

4. Verfahren nach Anspruch 3, **dadurch gekennzeichnet, daß** es eine erste Amplifikation umfaßt, bei der man als Primer die durch die allgemeinen Sequenzen EH1 und EH2 definierten Oligonukleotide oder Oligonukleotidmischungen verwendet, und eine zweite Amplifikation, bei der man als Matrize das Amplifikationsprodukt des ersten Schritts und als Primer die durch die allgemeinen Sequenzen EH2 und EH3 oder EH2 und EH4 definierten Oligonukleotide oder Oligonukleotidmischungen verwendet.

5. Verfahren nach Anspruch 3, **dadurch gekennzeichnet, daß** es einen Amplifikationsschritt umfaßt, bei dem man als Primer die durch die allgemeinen Sequenzen EH1 und EH2 definierten Oligonukleotide oder Oligonukleotidmischungen. verwendet, und einen Capture-Schritt für das Amplifikationsprodukt auf einem festen Träger, bei dem man als Capture-Sonde, vorzugsweise fixiert auf diesem festen Träger, wenigstens eines der Oligonukleotide oder eine der Oligonukleotidmischungen verwendet, die durch die allgemeinen Sequenzen EH3 und EH4 definiert sind.

6. Verfahren nach Anspruch 3, **dadurch gekennzeichnet, daß** es einen Amplifikationsschritt umfaßt, bei dem man als Primer die durch die allgemeinen Sequenzen EH1 und EH2 definierten Oligonukleotide oder Oligonukleotidmischungen verwendet, und einen Detektionsschritt für das Amplifikationsprodukt, bei dem man als Sonde wenigstens eines der Oligonukleotide oder eine der Oligonukleotidmischungen verwendet, die durch die allgemeinen Sequenzen EH3 und EH4 definiert sind.

7. Verfahren nach Anspruch 3, **dadurch gekennzeichnet, daß**:
- als Primer ein Oligonukleotid oder eine Oligonukleotidmischung, definiert durch die allgemeine Sequenz EH2, und ein Oligonukleotid oder eine Oligonukleotidmischung, definiert durch die allgemeine Sequenz EH1, verwendet werden, um ein 550bp-Amplifikationsfragment zu erhalten; oder
- als Primer ein Oligonukleotid oder eine Oligonukleotidmischung, definiert durch die allgemeine Sequenz EH2, und ein Oligonukleotid oder eine Oligonukleotidmischung, definiert durch die allgemeine Sequenz EH3, verwendet werden, um ein 450bp-Amplifikationsfragment zu erhalten; oder
- als Primer ein Oligonukleotid oder eine Oligonukleotidmischung, definiert durch die allgemeine Sequenz EH2, und ein Oligonukleotid oder eine Oligonukleotidmischung, definiert durch die allgemeine Sequenz EH4, verwendet werden, um ein 320bp-Amplifikationsfragment zu erhalten.

8. Verfahren zur Herstellung eines Plasmids, **dadurch gekennzeichnet, daß** es umfaßt:
- Durchführen eines Verfahrens nach Anspruch 7,
- Insertion des erhaltenen Amplifikationsfragments in ein Wirtsplasmid.

9. Verwendung wenigstens eines Oligonukleotids oder einer Oligonukleotidmischung nach Anspruch 1 oder eines Primerpaares nach Anspruch 2 zum Nachweis von Verotoxin-produzierenden *E. coli*-Stämmen.

10. Verwendung wenigstens eines Oligonukleotids oder einer Oligonukleotidmischung nach Anspruch 1 oder eines Primerpaares nach Anspruch 2 zur Suche nach Verotoxin-produzierenden *E. coli*-Stämmen in Nahrungsmitteln.

11. Verwendung wenigstens eines Oligonukleotids oder einer Oligonukleotidmischung nach Anspruch 1 oder eines Primerpaares nach Anspruch 2 zur Suche nach Verotoxin-produzierenden *E. coli*-Stämmen in einer Fäkalienprobe.

## Claims

1. Oligonucleotide or mixture of oligonucleotides usable as primers for the detection of verotoxic bacteria by gene amplification of vts genes, **characterised in that** they are chosen from amongst:
- an oligonucleotide, or a mixture of identical or different oligonucleotides, defined by the following general sequence EH1 (SEQ ID No. 1):
- an oligonucleotide, or a mixture of identical or different oligonucleotides, defined by the following general sequence EH2 (SEQ ID No. 2):
- an oligonucleotide, or a mixture of identical or different oligonucleotides, defined by the following general sequence EH3 (SEQ ID No. 3):
in which S represents G or C, W represents A or T, Y represents T or C, R represents A or G, K represents G or T, and H represents A, C or T.

2. Primer pair for the gene amplification of *vts* genes, **characterised in that** it is constituted by an oligonucleotide or a mixture of oligonucleotides of general sequence SEQ ID No. 2 (EH2) with an oligonucleotide or a mixture of oligonucleotides chosen from amongst:
- an oligonucleotide or a mixture of oligonucleotides of general sequence SEQ ID No. 1 (EH1);
- an oligonucleotide or a mixture of oligonucleotides of general sequence SEQ ID No. 3 (EH3);
- an oligonucleotide, or a mixture of identical or different oligonucleotides, defined by the following general sequence EH4 (SEQ ID No. 4):
in which S represents G or C, W represents A or T, Y represents T or C, R represents A or G, K represents G or T, and H represents A, C or T.

3. Method of gene amplification of a portion of DNA common to the VT1, VT2 and VTE genes of verotoxic bacteria, **characterised in that** the following are used as primers:
- an oligonucleotide or a mixture of oligonucleotides defined by the general sequence EH2 (SEQ ID No. 2), and
- an oligonucleotide or a mixture of oligonucleotides defined by one of the general sequences EH1 (SEQ ID No. 1), EH3 (SEQ ID No. 3) or EH4 (SEQ ID No. 4).

4. Method according to Claim 3, **characterised in that** it comprises a first amplification during which there are used, as primers, the oligonucleotides or mixtures of oligonucleotides defined by the general sequences EH1 and EH2, and a second amplification, during which there are used, as a template, the amplification product of the first step, and, as primers, the oligonucleotides or mixtures of oligonucleotides defined by the general sequences EH2 and EH3 or EH2 and EH4.

5. Method according to Claim 3, **characterised in that** it comprises an amplification step during which there are used, as primers, the oligonucleotides or mixtures of oligonucleotides defined by the general sequences EH1 and EH2, and a step of capture of the amplification product on a solid support, in which there is used, as a capture probe, at least one of the oligonucleotides or mixtures of oligonucleotides defined by the general sequences EH3 and EH4, previously fixed to said solid support.

6. Method according to Claim 3, **characterised in that** it comprises an amplification step during which there are used, as primers, the oligonucleotides or mixtures of oligonucleotides defined by the general sequences EH1 and EH2, and a step of detection of the amplification product, in which there is used, as a probe, at least one of the oligonucleotides or mixtures of oligonucleotides defined by the general sequences EH3 and EH4.

7. Method according to Claim 3, **characterised in that**:
- there are used, as primers, an oligonucleotide or a mixture of oligonucleotides defined by the general sequence EH2 and an oligonucleotide or a mixture of oligonucleotides defined by the general sequence EH1, in order to obtain an amplification fragment of 550 bp; or
- there are used, as primers, an oligonucleotide or a mixture of oligonucleotides defined by the general sequence EH2 and an oligonucleotide or a mixture of oligonucleotides defined by the general sequence EH3, in order to obtain an amplification fragment of 450 bp; or
- there are used, as primers, an oligonucleotide or a mixture of oligonucleotides defined by the general sequence EH2 and an oligonucleotide or a mixture of oligonucleotides defined by the general sequence EH4, in order to obtain an amplification fragment of 320 bp.

8. Method of preparing a plasmid, **characterised in that** it comprises:
- the use of a method according to Claim 7;
- insertion of the amplification fragment obtained into a host plasmid.

9. Use of at least one oligonucleotide or a mixture of oligonucleotides according to Claim 1, or a primer pair according to Claim 2, for detecting verotoxin-producing strains of *E. coli*.

10. Use of at least one oligonucleotide or a mixture of oligonucleotides according to Claim 1, or a primer pair according to Claim 2, for searching for verotoxin-producing strains of *E. coli,* in food products.

11. Use of at least one oligonucleotide or a mixture of oligonucleotides according to Claim 1, or a primer pair according to Claim 2, for searching for verotoxin-producing strains of *E. coli* in a sample of faeces.
